# EUROPEAN PATENT APPLICATION

(11) **EP 4 741 388 A1**
(43) Date of publication of application: **13.05.2026**
(21) Application number: 24835317.9
(22) Date of filing: 01.07.2024
(51) Int. Cl.: C07D 405/14, C07D 235/16, A61K 31/4545, A61K 31/4427, A61P 5/50, A61P 3/04, A61P 3/06, A61P 3/10, A61P 13/12

(54) **GLP1R AGONIST AND PREPARATION METHOD FOR INTERMEDIATE THEREOF**

(30) Priority: 04.07.2023 CN 202310809923; 13.07.2023 CN 202310859059; 02.01.2024 CN 202410003004
(71) Applicant: Hangzhou Zhongmeihuadong Pharmaceutical Co., Ltd., GongShu District HangZhou, Zhejiang 310011 (CN)
(72) Inventor: HAN, Mingliang, Hangzhou, Zhejiang 310011 (CN); GUAN, Tihong, Hangzhou, Zhejiang 310011 (CN); ZHENG, Junhao, Hangzhou, Zhejiang 310011 (CN); WANG, Pengfei, Hangzhou, Zhejiang 310011 (CN)
(74) Representative: Nederlandsch Octrooibureau
(86) International application number: PCT/CN2024/102861
(87) International publication number: WO 2025/007836

(57) **Abstract**

A GLPIR agonist and a preparation method for an intermediate thereof, and particularly, a preparation method for 2-(2-fluoro-4-(oxetane-3-yl)benzyl)oxy)-6-(piperidin-4-yl)pyridine and (S)-2-((4-(6-((2-fluoro-4-(oxetane-3-yl)methyl)oxy)pyridin-2-yl)piperidin-1-yl)methyl)-1-(oxetane-2-ylmethyl)-1H-benzimidazole-6-carboxylic acid. The preparation method has the advantages of low potential safety hazards, controllable cost, stable raw materials, and good yield, and is more suitable for industrial production.

## Description

### Technical Field

The present invention belongs to the technical field of drug synthesis, and specifically relates to methods for preparing 2-(2-fluoro-4-(oxetan-3-yl)benzyl)oxy)-6-(piperidin-4-yl)pyridine (Formula XI), an intermediate of a GLP-1 receptor agonist, and (S)-2-((4-(6-((2-fluoro-4-(oxetan-3-yl)methyl)oxy)pyridin-2-yl)piperidin-1-yl)methyl)-1-(oxetan-2-ylmethyl)-1H-benzoimidazole-6-carboxylic acid (Formula I).

### Background

(S)-2-((4-(6-((2-fluoro-4-(oxetan-3-yl)methyl)oxy)pyridin-2-yl)piperidin-1-yl)methyl)-1-(oxetan-2-ylmethyl)-1H-benzoimidazole-6-carboxylic acid (the compound of Formula I) is a non-peptide small-molecule glucagon-like peptide-1 receptor (GLP-1R) agonist with a molecular weight of 586.26 and a chemical structural formula as follows:

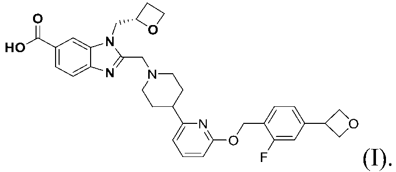

Patent of invention WO 2022/268152 A1 discloses for the first time the compound of Formula I, and a feasible preparation method thereof in which 3-(4-(bromomethyl)-3-fluorophenyl)oxetane and tert-butyl 4-(6-hydroxypyridin-2-yl)piperidine-1-carboxylate are used as raw materials, and subjected to nucleophilic substitution and deprotection of Boc to obtain Intermediate XI of the present invention (the specific steps are as follows) which then gives the compound of Formula I through substitution and hydrolysis reactions. Among others, 3-(4-(bromomethyl)-3-fluorophenyl)oxetane is obtained by bromination of a benzyl alcohol derivative, and tert-butyl 4-(6-hydroxypyridin-2-yl)piperidine-1-carboxylate is obtained by hydroxylation of a 6-chloropyridine derivative catalyzed by a palladium catalyst.

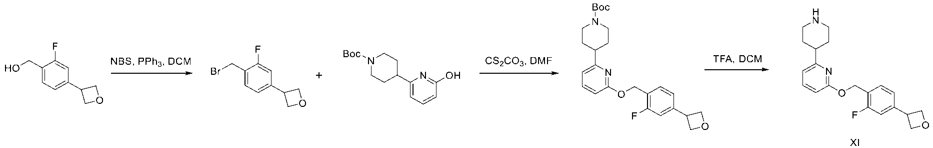

In kilogram order, scale-up synthesis tests on the above route, the raw material 3-(4-(bromomethyl)-3-fluorophenyl)oxetane is unstable and highly sensitized. Even under suitable protection, laboratory personnel still suffer from skin rashes or ulcers, which poses potential safety hazards in industrial production. In addition, the hydroxyl group at 6-position of tert-butyl 4-(6-hydroxypyridin-2-yl)piperidine-1-carboxylate needs to be obtained by conversion of a chloropyridine derivative in the presence of a noble metal palladium catalyst, resulting in high cost. Therefore, there is a great demand for developing a process route with stable raw materials, controllable cost and facile production.

### Summary of the Invention

To overcome the problems of unstable and highly sensitized raw materials or high cost mentioned in the Background, the present invention provides a novel method for preparing 2-(2-fluoro-4-(oxetan-3-yl)benzyl)oxy)-6-(piperidin-4-yl)pyridine (Formula XI). This method overcomes the instability and potential safety hazards of 3-(4-(bromomethyl)-3-fluorophenyl)oxetane, effectively simplifies the steps and reduces the cost of raw materials, the process of which is as follows: 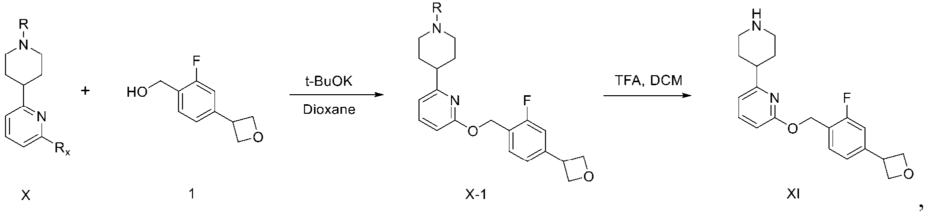 wherein R is an amino protecting group independently selected from one of benzyloxycarbonyl (Cbz), tert-butyloxycarbonyl (Boc), p-methoxybenzyl (PMB), benzyl (Bn), trityl (Trt), p-toluenesulfonyl (Tos), phthaloyl (Pht) and allyloxycarbonyl (Alloc); wherein Rx is selected from the group consisting of Cl, Br and I, for example, Cl.

In the method provided in the present invention, Compound 1 and a 6-halopyridine derivative are used as raw materials to generate Compound X-1 through a nucleophilic substitution reaction and give the compound of Formula XI after removing the amino protecting group. Then, the compound of Formula I is obtained from the compound of Formula XI through steps such as substitution and hydrolysis.

### Detailed Description of the Invention

The present invention provides a novel method for preparing 2-(2-fluoro-4-(oxetan-3-yl)benzyl)oxy)-6-(piperidin-4-yl)pyridine (Formula XI), comprising the following steps:
a-1) dissolving Compound X in an organic solvent, adding Compound 1 to react in the presence of a base to obtain Compound X-1: 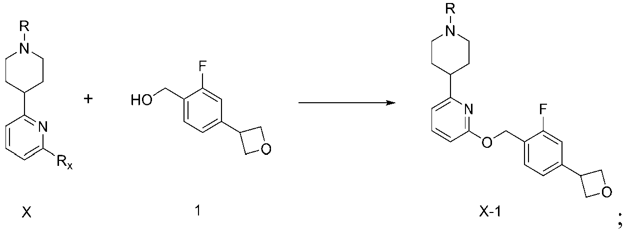
a-2) removing the amino protecting group R using a suitable solvent and suitable reaction conditions to obtain Compound XI: 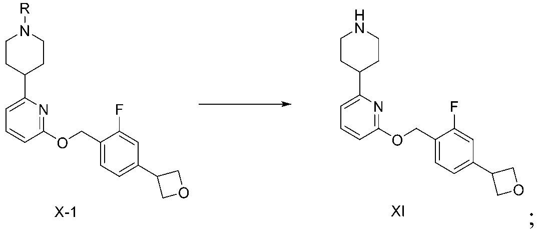 wherein said R is independently selected from the group consisting of benzyloxycarbonyl, tert-butyloxycarbonyl, p-methoxybenzyl, benzyl, trityl, p-toluenesulfonyl, phthaloyl and allyloxycarbonyl; and said Rx is selected from the group consisting of Cl, Br and I, for example, Cl.

In some embodiments, said R is independently selected from the group consisting of benzyloxycarbonyl, tert-butyloxycarbonyl, p-methoxybenzyl, benzyl, trityl, p-toluenesulfonyl, phthaloyl and allyloxycarbonyl, preferably tert-butyloxycarbonyl.

In some embodiments, the organic solvent used in Step a-1) is an aprotic solvent selected from the group consisting of dioxane, N,N-dimethyl formamide, N,N-dimethylacetamide, dimethyl sulfoxide, acetonitrile, toluene, tetrahydrofuran, pyridine and 2-methyl tetrahydrofuran, preferably dioxane, or toluene.

In some embodiments, the base used in Step a-1) is selected from the group consisting of sodium hydroxide, sodium bicarbonate, lithium hydroxide, potassium carbonate, potassium bicarbonate, potassium hydroxide, sodium methoxide, sodium tert-butoxide, potassium tert-butoxide, sodium ethoxide, n-butyl lithium, lithium diisopropylamide (LDA) and lithium hexamethyldisilazide (LiHMDS), preferably potassium tert-butoxide, or potassium hydroxide.

In some embodiments, the molar feed ratio of the base to Compound 1 used in Step a-1) is 1:1 ~ 4:1, preferably 1:1 ~3:1.

In some embodiments, the molar feed ratio of Compound X to Compound 1 in Step a-1) is 3:1 ~ 1:1.

In some embodiments, after adding Compound X and Compound 1, the temperature can be controlled at T1 and then reduced to room temperature in Step a-1).

In some embodiments, said T1 in Step a-1) is selected from 30 ~ 130°C, preferably 60 ~ 100°C, for example, 80 ~ 100°C.

In some embodiments, the solvent used in Step a-2) is selected from the group consisting of dichloromethane, methanol, tetrahydrofuran, ethyl acetate, chloroform, acetonitrile and DMF, preferably dichloromethane.

In some embodiments, the reaction temperature in Step a-2) is selected from -20 to 50°C.

In some embodiments, after the completion of the reaction in Step a-1), post-treatment can be optionally performed, and the post-treatment includes one or more of extraction, washing, drying, concentration and filtration.

In some embodiments, after the completion of the reaction in Step a-2), post-treatment can be optionally performed, and the post-treatment includes one or more of extraction, washing, drying, concentration and filtration.

As a specific embodiment, Step a-1) can be carried out as follows: dissolving Compound X in an organic solvent, then adding a base, stirring and mixing, then adding Compound 1, controlling the temperature at 25 ~ 130°C and reacting for 1 ~ 20 h, reducing the temperature to room temperature, adding an extraction solvent and water to the reaction solution for extraction, separating the aqueous layer, washing the organic layer with purified water or brine, drying the organic layer over anhydrous sodium sulfate, filtering by suction, concentrating the filtrate to no distillate, then slurrying, placing the filter cake at 35 ~ 45°C for drying under reduced pressure for 10 ~ 12 h to obtain Intermediate X-1.

As a specific embodiment, when R is tert-butyloxycarbonyl (Boc), a process route of the present invention is as follows:

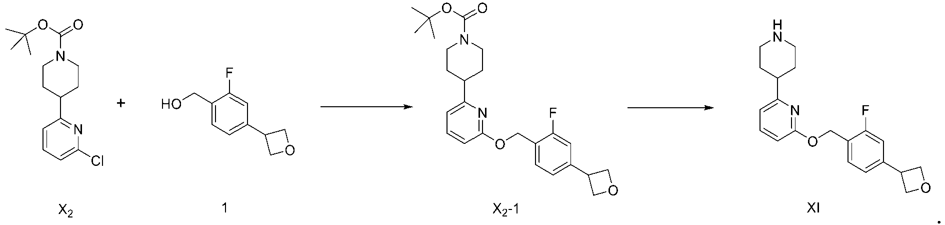

As a specific embodiment, when R is benzyl (Bn), a process route of the present invention is as follows:

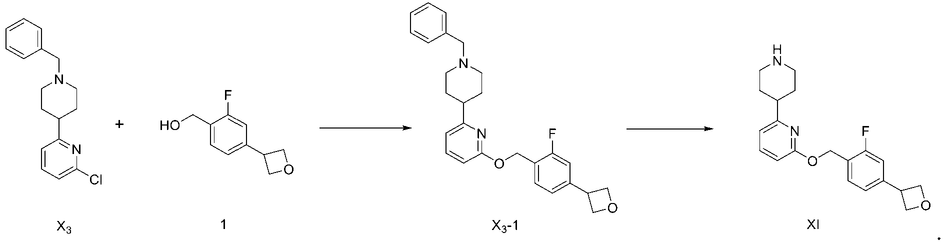

The beneficial effects of these technical solutions are as follows: Compound 1 can be stored stably at room temperature without sensitization; and Compound X (including Compound X₂ and Compound X₃) has no hydroxyl substitution, which avoids the use of noble metal palladium catalysts, effectively reduces the raw material costs and improves the yield of Compound XI.

The present invention further provides a method for preparing the compound of Formula I from Compound XI, comprising the following steps:
a-3) dissolving Compound XI in an organic solvent, adding a base and Compound V to react to obtain Compound VI: 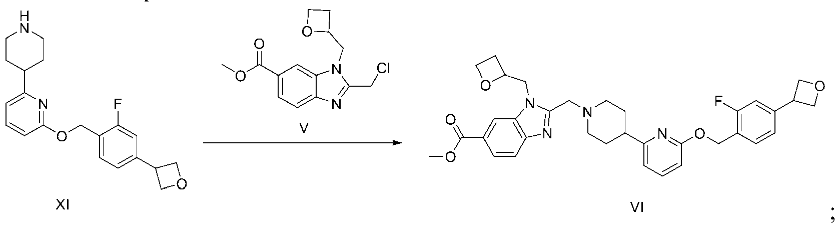
a-4) dissolving Compound VI in an organic solvent, adding a base to react to obtain the Compound I:

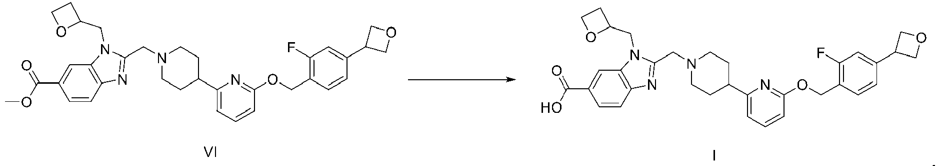

In some embodiments, the organic solvent used in Step a-3) is selected from the group consisting of acetonitrile, N,N-dimethyl formamide, dimethyl sulfoxide, tetrahydrofuran, ethyl acetate, N,N-dimethyl formamide and n-hexane, preferably N,N-dimethyl formamide.

In some embodiments, the molar feed ratio of the compound of Formula XI to the compound of Formula V in Step a-3) is 1:1 ~ 2:1.

In some embodiments, the base used in Step a-3) is selected from the group consisting of sodium hydroxide, lithium hydroxide, sodium bicarbonate, potassium carbonate, potassium bicarbonate, potassium hydroxide, sodium methoxide, sodium tert-butoxide, potassium tert-butoxide, sodium ethoxide, n-butyl lithium, lithium diisopropylamide (LDA) and lithium hexamethyldisilazide (LiHMDS), preferably potassium tert-butoxide, sodium bicarbonate, or potassium carbonate.

In some embodiments, the molar ratio of the base to Compound XI used in Step a-3) is 1:1 ~ 3:1.

In some embodiments, the organic solvent used in Step a-4) is one or more selected from the group consisting of methanol, ethanol, isopropanol, n-butanol and tert-butanol, preferably methanol.

In some embodiments, the base used in Step a-4) is selected from the group consisting of sodium hydroxide, lithium hydroxide, sodium bicarbonate, potassium carbonate, potassium bicarbonate, potassium hydroxide, sodium methoxide, sodium tert-butoxide, potassium tert-butoxide, sodium ethoxide, n-butyl lithium, lithium diisopropylamide (LDA) and lithium hexamethyldisilazide (LiHMDS), preferably potassium tert-butoxide, sodium bicarbonate, or lithium hydroxide.

In some embodiments, the molar ratio of the base to Compound VI used in Step a-4) is 1:1 ~ 5:1.

In some embodiments, after the completion of the reaction in Step a-3), post-treatment can be optionally performed, and the post-treatment includes one or more of extraction, washing, drying, concentration and filtration.

In some embodiments, after the completion of the reaction in Step a-4), post-treatment can be optionally performed, and the post-treatment includes one or more of extraction, washing, drying, concentration and filtration.

As a specific embodiment, Step a-3) can be carried out as follows: dissolving Compound XI in an organic solvent with stirring until completely dissolved, sequentially adding a base and Compound V to react with stirring at 20 ~ 100°C for 0.5 ~ 18 h under a nitrogen atmosphere, pouring dropwise the reaction solution into water, and collecting the precipitated solid. The solid is dissolved with an organic solvent, then washed with brine or purified water, and the organic layer is dried over anhydrous sodium sulfate; the filtrate is concentrated to no distillate, then slurried with methyl tert-butyl ether. The filter cake is placed at 35 ~ 45°C for drying under reduced pressure for 1 ~ 12 h to obtain Compound VI.

As a specific embodiment, Step a-4) can be carried out as follows: dissolving Compound VI in an organic solvent, adding an aqueous solution of lithium hydroxide with stirring, reacting at 0 ~ 60°C for 0.5 ~ 24 h under a nitrogen atmosphere, diluting the reaction solution by adding water, then washing the aqueous phase with an organic solvent, separating the organic phase, adjusting the pH of the aqueous phase to acidic, adding an organic solvent for extraction, combining the organic phases, adding n-heptane to the organic phase, collecting the precipitated solid, then slurrying with methyl tert-butyl ether. The filter cake is placed at 35 ~ 45°C for drying under reduced pressure for 1 ~ 12 h to obtain the target compound of Formula I.

The beneficial effects of the technical solutions of the invention are as follows:
1. overcoming the potential hazards of the instability and high sensitization of 3-(4-(bromomethyl)-3-fluorophenyl)oxetane which is used as a raw material in the prior art, avoiding the use of noble metal palladium catalysts, and significantly reducing the costs, thereby obtaining the process route with stable raw materials, controllable costs and applicable to industrial scale-up as described in the present application;
2. increasing the yields of Compound VI (increased from 22.0% in the prior art to 80% or more) and Compound I (increased from 18.6% in the prior art to 90% or more) by optimizing reaction solvent systems and reaction conditions and using suitable bases; and
3. avoiding the use of column chromatography, being able to reach the required product purity for each of the intermediate and the product through recrystallization or slurrying in the process routes according the present invention, with a purity of the intermediate of more than 98% and a purity of the product of more than 99%.

### Brief Description of the Drawings

Figure 1: the ¹H NMR spectrum of Compound X₂-1.
Figure 2: the ¹H NMR spectrum of Compound X₃-1.
Figure 3: the ¹H NMR spectrum of Compound XI.
Figure 4: the ¹H NMR spectrum of Compound VI.
Figure 5: the ¹H NMR spectrum of Compound I.
Figure 6: the HPLC chromatogram of Compound X₂-1.
Figure 7: the HPLC chromatogram of Compound XI.
Figure 8: the HPLC chromatogram of Compound VI.
Figure 9: the HPLC chromatogram of Compound I.

### Examples

The experimental methods without specific conditions noted in the Examples of the present invention generally employ conventional conditions or the conditions recommended by manufacturers of raw materials or commodities; and reagents without indicated sources are generally conventional reagents which are commercially available, or can be prepared from known reagents through conventional methods.

The present invention will be further described in detail below in conjunction with specific Examples.

### Example 1.1: Preparation of Compound X-1 (i.e., the preparation of Compound X₂-1 when R is Boc)

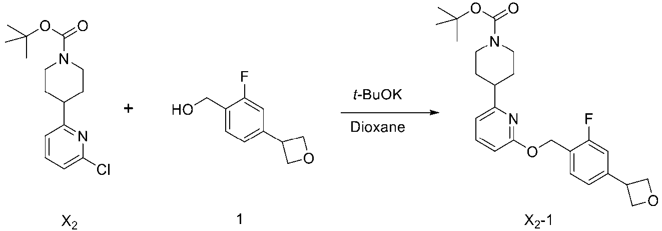

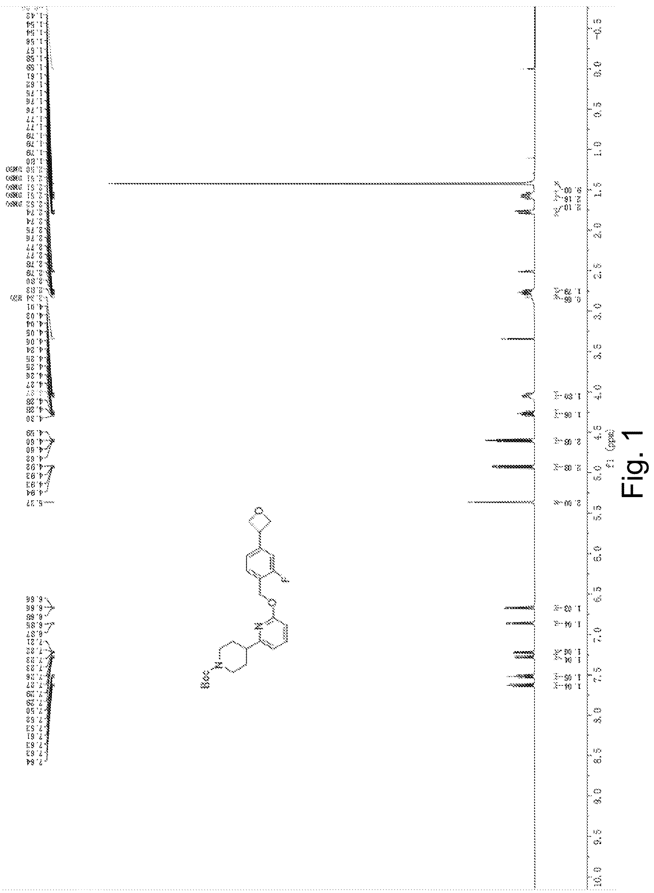

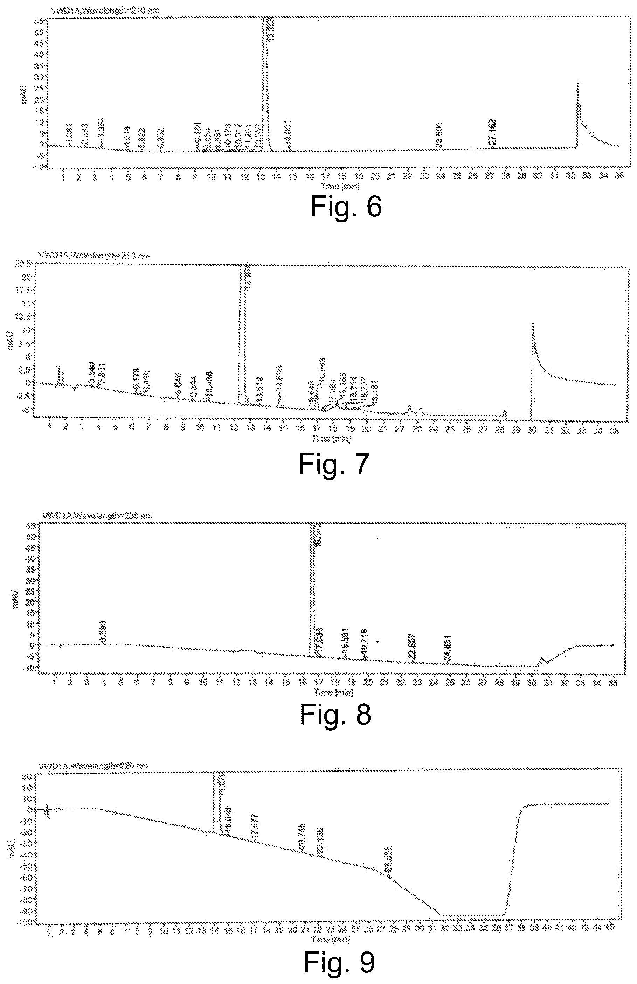

120 mL of 1,4-dioxane, 18 g of Compound X₂, 10 g of Compound 1 and 9.3 g of potassium tert-butoxide were added to a reaction flask in sequence under nitrogen protection, heated up to 100°C and then stirred for reaction for 18 hours. After cooling to room temperature, 150 mL of ethyl acetate was added to dilute the reaction solution, then 200 mL of purified water was added, and stirred for 25 min, and allowed to stand for layer separation. The organic phase was collected, and the aqueous phase was back-extracted once with 150 mL of ethyl acetate. The organic phases were combined, and washed with purified water and saturated brine separately (2×200 mL each), and the organic phase was collected. The organic phase was dried over anhydrous sodium sulfate, filtered, and concentrated to dryness. The concentrated dry crude product was added with 50 mL of ethyl acetate to dissolve, stirred for 1 h, then filtered to remove insoluble substances. 100 mL of n-hexane was added to the filtrate for dilution. The organic phase above was poured into a pre-packed silica gel column, and the eluent was collected. The collected eluent was concentrated to dryness, added with a mixed solvent of methyl tert-butyl ether and n-hexane (V/V = 1:20) and heated to dissolve completely, then cooled to room temperature for crystallization, and filtered by suction. The filter cake was rinsed with n-hexane, and dried in vacuum to obtain Compound X₂-1 (15.0 g in total, a molar yield of 61%, a purity of greater than 98.5%). The ¹H NMR spectrum of Compound X₂-1 is shown in Figure 1, and the HPLC chromatogram of X₂-1 is shown in Figure 6.

¹H NMR (500 MHz, chloroform-d) δ 7.59 - 7.39 (m, 2H), 7.18 - 7.11 (m, 2H), 6.72 (d, *J* = 7.2 Hz, 1H), 6.62 (d, *J =* 8.2 Hz, 1H), 5.43 (s, 2H), 5.07 (dd, *J* = 8.3, 6.1 Hz, 2H), 4.73 (t, *J* = 6.3 Hz, 2H), 4.21 (ddd, *J* = 15.0, 8.3, 6.6 Hz, 2H), 2.85 (d, *J* = 13.5 Hz, 2H), 2.74 (ddd, *J* = 11.8, 8.1, 3.7 Hz, 1H), 1.93 - 1.82 (m, 2H), 1.73 (qd, *J* = 12.6, 4.4 Hz, 2H), 1.49 (s, 9H).

HRMS (ESI): Calculated [M+H]⁺ 443.2341, Found 443.2349.

### Example 1.2: Preparation of Compound X-1 (i.e., the preparation of Compound X₂-1 when R is Boc)

120 mL of 1,4-dioxane, 18 g of Compound X₂, 10 g of Compound 1 and 9.3 g of potassium tert-butoxide were added to a reaction flask in sequence under nitrogen protection, heated up to 65°C and then stirred for reaction for 7 hours. After cooling to room temperature, 150 mL of ethyl acetate was added to dilute the reaction solution, then 200 mL of purified water was added, and stirred for 25 min, and allowed to stand for layer separation. The organic phase was collected, and the aqueous phase was back-extracted once with 150 mL of ethyl acetate. The organic phases were combined, and washed with purified water and saturated brine separately (2×200 mL each), and the organic phase was collected. The organic phase was dried over anhydrous sodium sulfate, filtered, and concentrated to dryness. The concentrated dry crude product was added with 150 mL of a mixed solvent of ethyl acetate and n-hexane (V/V = 1:2) to dissolve. The organic phase above was filtered through a suction funnel filled with silica gel (about 30 g), and the filter cake was rinsed with a mixed solvent of n-hexane and ethyl acetate (V/V = 1:1). The filtrate and rinse solution were combined, concentrated to dryness, then added with a mixed solvent of methyl tert-butyl ether and n-hexane (V/V = 1:4) and heated to dissolve completely, then cooled to room temperature for crystallization, and filtered by suction. The filter cake was rinsed with n-hexane, and dried in vacuum to obtain Compound X₂-1 (16.4 g, a molar yield of 67%, a purity of greater than 98.5%).

### Example 2.1: Preparation of Compound XI (preparation of Compound XI when R is Boc)

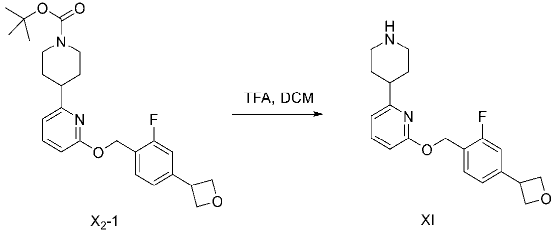

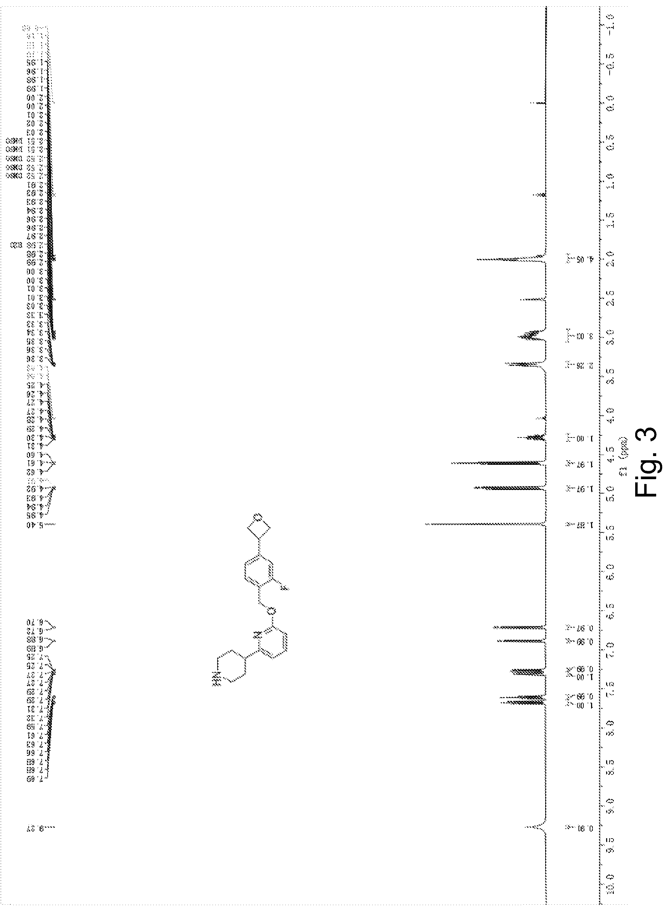

100 mL of dichloromethane and 10 g of Compound X₂-1 were added to a reaction flask in sequence under nitrogen protection, stirred to dissolve completely, then added with 20 mL of trifluoroacetic acid dropwise at -10°C. The temperature of the reaction solution was controlled below -5°C during the dropwise addition, and the dropwise addition was complete within 30 min. Then, the reaction was conducted at -5°C for 6 hours. The reaction solution was slowly poured into 800 mL of saturated aqueous sodium bicarbonate solution, the aqueous layer was separated out, and the organic layer was retained. The organic phase was washed with 10% aqueous sodium chloride solution (3×100 mL). The organic phase was dried over anhydrous sodium sulfate, filtered and concentrated to dryness. The concentrated dry crude product was slurried with ethyl acetate at 80°C for 2 h, then cooled to room temperature, and filtered by suction. The filter cake was rinsed with ethyl acetate, and dried in vacuum to obtain Compound XI (6.5 g, a molar yield of 84%, a purity of greater than 99%). The ¹H NMR spectrum of Compound XI is shown in Figure 3, and the HPLC chromatogram of Compound XI is shown in Figure 7.

¹H NMR (500 MHz, DMSO-d6) δ 9.23 (s, 1H), 7.68 (dd, J = 8.2, 7.3 Hz, 1H), 7.61 (t, J = 7.9 Hz, 1H), 7.33 - 7.23 (m, 2H), 6.88 (d, J = 7.3 Hz, 1H), 6.71 (d, J = 8.2 Hz, 1H), 5.39 (s, 2H), 4.93 (dd, J = 8.3, 6.0 Hz, 2H), 4.61 (dd, J = 6.7, 6.0 Hz, 2H), 4.28 (ddd, J = 15.0, 8.4, 6.8 Hz, 1H), 3.34 (dt, J = 12.7, 3.5 Hz, 2H), 3.06 - 2.88 (m, 3H), 2.06 - 1.96 (m, 4H).

HRMS (ESI): Calculated [M+H]⁺ 343.1817, Found 343.1828.

### Example 2.2: Preparation of Compound XI (preparation of Compound XI when R is Boc)

90 mL of dichloromethane and 30 mL of trifluoroacetic acid were added to a reaction flask in sequence under nitrogen protection, and 10 g of Compound X₂-1 was added to the reaction flask after cooling to -10°C. The temperature of the reaction solution was controlled at -5°C to 0°C, and the reaction was conducted with stirring. The reaction progress was monitored by TLC, and the reaction was stopped after about 45 min. The reaction solution was slowly poured into 800 mL of saturated aqueous sodium carbonate solution. After stirring for 30 min, the aqueous layer was separated out. The organic phase was washed with 20% aqueous ammonium chloride solution (4×100 mL). The organic phase was dried over anhydrous sodium sulfate, filtered and concentrated to dryness. The concentrated dry crude product was slurried with ethyl acetate at 80°C for 2 h, then cooled to room temperature, and filtered by suction. The filter cake was rinsed with ethyl acetate, and dried in vacuum to obtain Compound XI (6.5 g, a molar yield of 84%, a purity of greater than 99%).

### Example 3.1: Preparation of Compound X₃-1 (preparation of Compound X₃-1 when R is Bn):

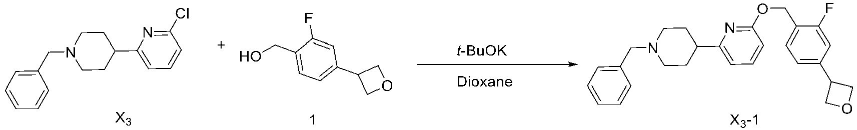

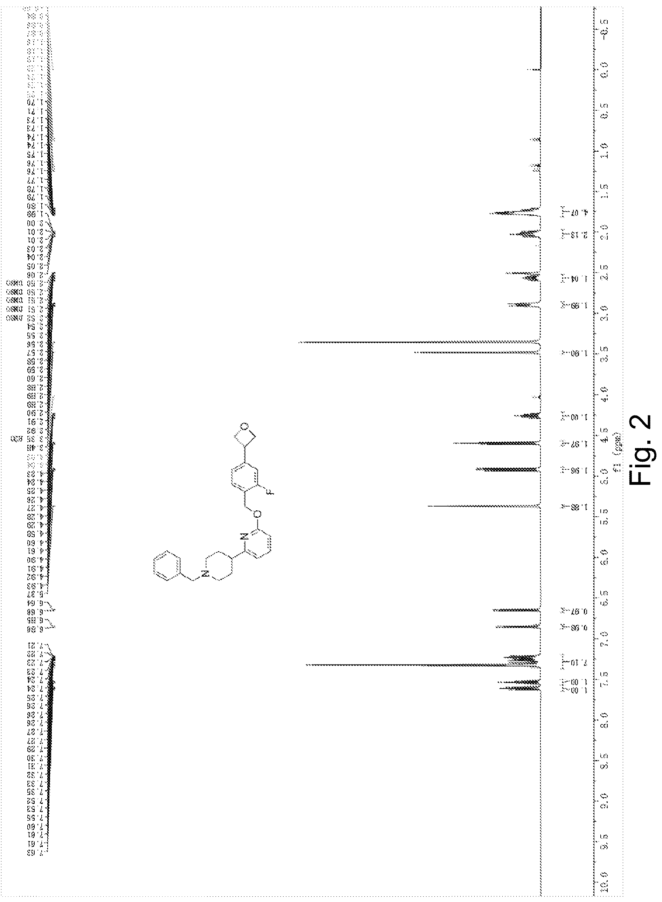

120 mL of 1,4-dioxane, 17.4 g of Compound X₃, 10 g of Compound 1 and 9.3 g of potassium tert-butoxide were added to a reaction flask in sequence under nitrogen protection, heated up to 100°C and then stirred for reaction for 18 hours. After cooling to room temperature, 150 mL of ethyl acetate was added to dilute the reaction solution, then added with 200 mL of purified water, stirred for 25 min, and allowed to stand for layer separation. The organic phase was collected, and the aqueous phase was back-extracted once with 150 mL of ethyl acetate. The combined organic phases were washed with purified water and saturated brine separately (2×200 mL each), and the organic phase was collected. The organic phase was dried over anhydrous sodium sulfate, filtered and concentrated to dryness. The concentrated dry crude product was added with 50 mL of ethyl acetate to dissolve, stirred for 1 h, then filtered to remove insoluble substances. 100 mL of n-hexane was added to the filtrate for dilution. The organic phase above was poured into a pre-packed silica gel column, and the eluent was collected. The collected eluent was concentrated to dryness, added with a mixed solvent of methyl tert-butyl ether and n-hexane (V/V = 1:20) and heated to dissolve completely, then cooled to room temperature for crystallization, and filtered by suction. The filter cake was rinsed with n-hexane, and dried in vacuum to obtain Compound X₃-1 (18.8 g, a molar yield of 78.9%). The ¹H NMR spectrum of Compound X₃-1 is shown in Figure 2.

¹H NMR (500 MHz, DMSO-*d6*) δ 7.61 (dd, J = 8.2, 7.3 Hz, 1H), 7.53 (t, J = 7.8 Hz, 1H), 7.36 - 7.27 (m, 5H), 7.27 - 7.20 (m, 2H), 6.85 (d, J = 7.3 Hz, 1H), 6.65 (d, J = 8.1 Hz, 1H), 5.37 (s, 2H), 4.92 (dd, J = 8.4, 5.9 Hz, 2H), 4.60 (t, J = 6.3 Hz, 2H), 4.26 (ddd, J = 15.1, 8.3, 6.7 Hz, 1H), 3.48 (s, 2H), 2.90 (dt, J = 11.8, 3.4 Hz, 2H), 2.57 (tt, J = 10.1, 5.0 Hz, 1H), 2.09 - 1.95 (m, 2H), 1.82 - 1.69 (m, 4H).

HRMS (ESI): Calculated [M+H]⁺ 433.2286, Found 433.2275.

### Example 3.2: Preparation of Compound X₃-1 (preparation of Compound X₃-1 when R is Bn):

120 mL of 1,4-dioxane, 17.4 g of Compound X₃, 10 g of Compound 1 and 9.3 g of potassium tert-butoxide were added to a reaction flask in sequence under nitrogen protection, heated up to 65°C and then stirred for reaction for 7 hours. After cooling to room temperature, 150 mL of ethyl acetate was added to dilute the reaction solution, then 200 mL of purified water was added, and stirred for 25 min, and allowed to stand for layer separation. The organic phase was collected, and the aqueous phase was back-extracted once with 150 mL of ethyl acetate. The organic phases were combined, and washed with purified water and saturated brine separately (2×200 mL each), and the organic phase was collected. The organic phase was dried over anhydrous sodium sulfate, filtered, and concentrated to dryness. The concentrated dry crude product was added with 150 mL of a mixed solvent of ethyl acetate and n-hexane (V/V = 1:2) to dissolve. The organic phase above was filtered through a suction funnel filled with silica gel (about 30 g), and the filter cake was rinsed with a mixed solvent of n-hexane and ethyl acetate (V/V = 1:2). The filtrate and rinse solution were combined, concentrated to dryness, then added with a mixed solvent of methyl tert-butyl ether and n-hexane (V/V = 1:4) and heated to dissolve completely, then cooled to room temperature for crystallization, and filtered by suction. The filter cake was rinsed with n-hexane, and dried in vacuum to obtain Compound X₃-1.

### Example 4: Preparation of Compound XI (preparation of Compound XI when R is Bn)

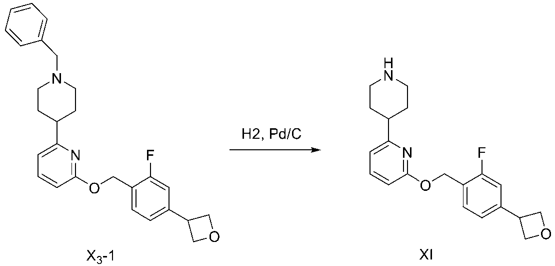

10 g of Compound X₃-1 was dissolved in 100 mL of anhydrous methanol, and then added with 1 g of 10% Pd/C, and hydrogen gas (1 atm) was introduced. The reaction was conducted with stirring at 45 °C for 12 hours. Pd/C was removed by filtration under reduced pressure, and the filtrate was concentrated to dryness. The concentrated crude product was slurried with ethyl acetate at 80 °C for 2 hours, then cooled to room temperature and subjected to suction filtration. The filter cake was rinsed with ethyl acetate and vacuum-dried to obtain compound XI (7.2 g, a molar yield of 91%, a purity of greater than 99%).

¹H NMR (500 MHz, DMSO-d6) δ 9.23 (s, 1H), 7.68 (dd, J = 8.2, 7.3 Hz, 1H), 7.61 (t, J = 7.9 Hz, 1H), 7.33 - 7.23 (m, 2H), 6.88 (d, J = 7.3 Hz, 1H), 6.71 (d, J = 8.2 Hz, 1H), 5.39 (s, 2H), 4.93 (dd, J = 8.3, 6.0 Hz, 2H), 4.61 (dd, J = 6.7, 6.0 Hz, 2H), 4.28 (ddd, J = 15.0, 8.4, 6.8 Hz, 1H), 3.34 (dt, J = 12.7, 3.5 Hz, 2H), 3.06 - 2.88 (m, 3H), 2.06 - 1.96 (m, 4H).

HRMS (ESI): Calculated [M+H]⁺ 343.1817, Found 343.1828.

### Example 5: Preparation of the compound of Formula I

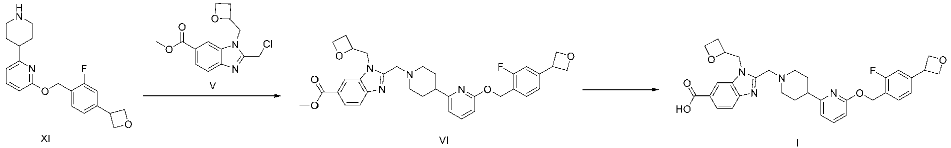

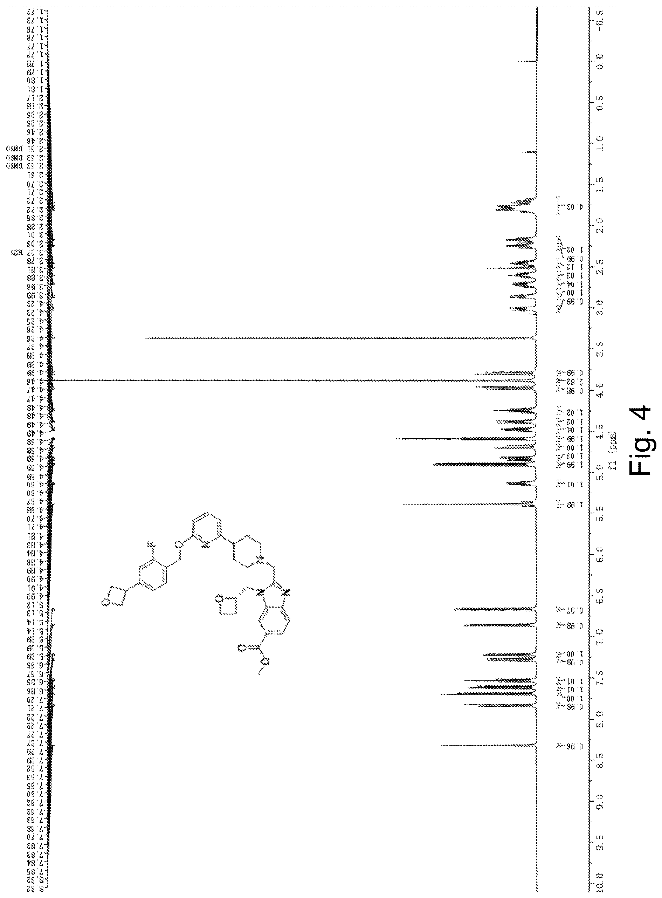

60 mL of DMF and 6.0 g of Compound XI were added to a reaction flask under nitrogen protection and stirred to dissolve completely. Subsequently, 3.5 g of K₂CO₃ and 4.5 g of Compound V were added sequentially, stirred at room temperature for 60 minutes and then heated to 50 °C for a 6 h reaction. After the reaction solution was cooled to room temperature, 360 mL of purified water was added, and stirred for 2 hours before suction filtration. The filter cake was dissolved in 120 mL of ethyl acetate, and the organic phase was washed twice with 60 mL of saturated brine. The organic phase was dried over anhydrous sodium sulfate, filtered, and concentrated to dryness. The concentrated dry crude product was slurried with 60 mL of methyl tert-butyl ether at 50 °C for 2 h, then cooled to room temperature and subjected to suction filtration. The filter cake was rinsed with ethyl acetate and vacuum-dried to obtain 8.5 g of Compound VI (a molar yield of 81%, a purity of greater than 99%). The ¹H NMR spectrum of Compound VI is shown in Figure 4, and the HPLC chromatogram of Compound VI is shown in Figure 8.

¹H NMR (500 MHz, chloroform-d) δ 8.19 (d, *J =* 1.5 Hz, 1H), 7.97 (dd, *J =* 8.5, 1.6 Hz, 1H), 7.75 (d, *J =* 8.4 Hz, 1H), 7.49 (ddd, *J =* 9.5, 6.4, 1.8 Hz, 2H), 7.19-7.08 (m, 2H), 6.73 (d, *J =* 7.2 Hz, 1H), 6.61 (d, *J* = 8.1 Hz, 1H), 5.44 (s, 2H), 5.24 (dd, *J =* 7.0, 3.2 Hz, 1H), 5.06 (dd, *J =* 8.3, 6.0 Hz, 2H), 4.78 (d, 6.2 Hz, 1H), 4.74-4.68 (m, 3H), 4.62 (ddd, 8.3, 7.5, 6.0 Hz, 1H), 4.42 (dt, *J =* 9.1, 6.0 Hz, 1H), 4.24-4.14 (m, 1H), 3.94 (s, 3H), 3.01 (dd, *J =* 25.2, 11.0 Hz, 2H), 2.82-2.70 (m, 1H), 2.64 (dt, *J =* 11.1, 6.5 Hz, 1H), 2.56-2.44 (m, 1H), 2.43-2.21 (m, 1H), 1.88 (ddd, *J* = 21.9, 11.8, 6.0 Hz, 4H).

HRMS (ESI): Calculated [M+H]⁺ 601.2821, Found 601.2828.

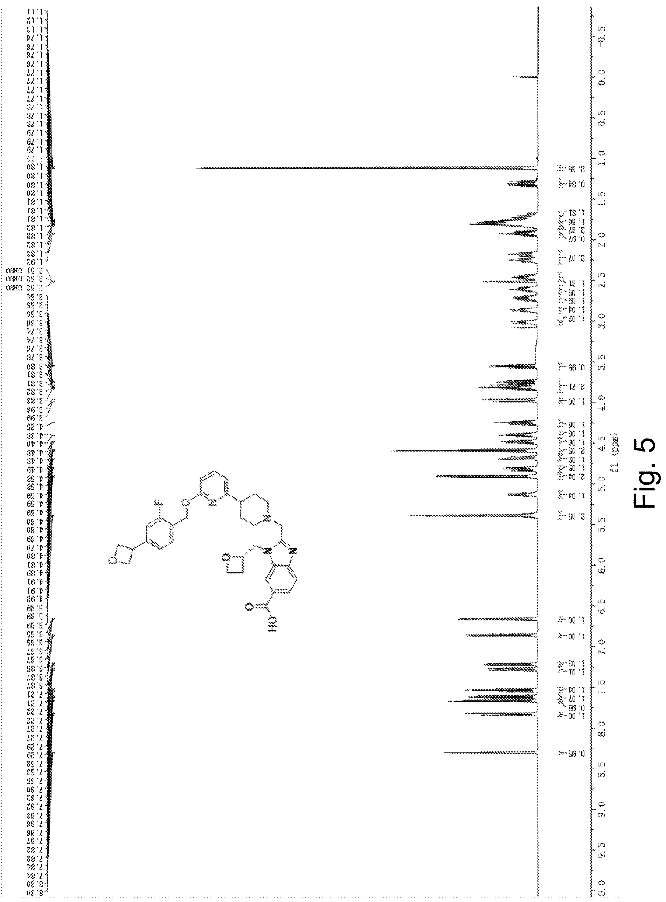

At room temperature, 8.0 g of Compound VI and 80 mL of methanol were added to a reaction flask. 1.12 g of lithium hydroxide monohydrate was dissolved in 10 mL of purified water, and then added to the reaction flask, stirred at room temperature for 1 h. The reaction solution was heated to 50 °C and stirred for 18 hours while maintaining the temperature. After the reaction solution was cooled to room temperature, 100 mL of purified water was added, and methanol was removed by concentration under reduced pressure. The aqueous phase was washed once with 40 mL of 2-methyltetrahydrofuran, and the aqueous phase was retained. Another 60 mL of 2-methyltetrahydrofuran was added, and acetic acid was added dropwise to adjust the pH of the system to 5.4-5.6. The layers were separated, and the organic phase was retained. The organic phase was washed with 50 mL of purified water, and n-heptane was added dropwise to the organic phase after layer separation. The precipitated solid was collected, and then hot-slurried with methyl tert-butyl ether at 50 °C for 4 h, cooled to room temperature, and then stirred for 4 h. After suction filtration, the filter cake was rinsed with methyl tert-butyl ether. The obtained solid was vacuum-dried at 45 °C to yield 7.1 g of Compound I (a molar yield of 91%, a purity of greater than 99%). The ¹H NMR spectrum of Compound I is shown in Figure 5, and the HPLC chromatogram of Compound I is shown in Figure 9.

¹H NMR (500 MHz, DMSO-*d6*) δ 12.81 (s, 1H), 8.29 (d, J = 1.5 Hz, 1H), 7.82 (dd, J = 8.4, 1.6 Hz, 1H), 7.69 - 7.58 (m, 2H), 7.53 (t, J = 7.8 Hz, 1H), 7.28 (dd, J = 11.3, 1.7 Hz, 1H), 7.22 (dd, J = 7.9, 1.7 Hz, 1H), 6.86 (d, J = 7.3 Hz, 1H), 6.66 (d, J = 8.1 Hz, 1H), 5.39 (d, J = 2.1 Hz, 2H), 5.13 (qd, J = 7.1, 2.8 Hz, 1H), 4.90 (dd, J = 8.4, 6.0 Hz, 2H), 4.82 (dd, J = 15.2, 7.3 Hz, 1H), 4.68 (dd, J = 15.2, 2.8 Hz, 1H), 4.59 (t, J = 6.3 Hz, 2H), 4.52 - 4.44 (m, 1H), 4.39 (dt, J = 9.0, 5.8 Hz, 1H), 4.25 (ddd, J = 15.1, 8.4, 6.8 Hz, 1H), 3.97 (d, J = 13.5 Hz, 1H), 3.79 (d, J = 13.5 Hz, 1H), 3.01 (dt, J = 11.4, 3.9 Hz, 1H), 2.86 (dt, J = 11.4, 4.0 Hz, 1H), 2.72 (dtd, J = 11.1, 8.1, 6.1 Hz, 1H), 2.61 (tt, J = 11.6, 4.2 Hz, 1H), 2.46 (ddt, J = 11.1, 8.9, 7.0 Hz, 1H), 2.22 (dtd, J = 35.9, 11.4, 2.9 Hz, 2H), 1.76 (dddd, J = 38.0, 20.7, 13.4, 10.7 Hz, 4H).

HRMS (ESI): Calculated [M+H]⁺ 587.2664, Found 587.2668.

### Example 6: Preparation of Compound X-1 (preparation of Compound X₂-1 when R is Boc)

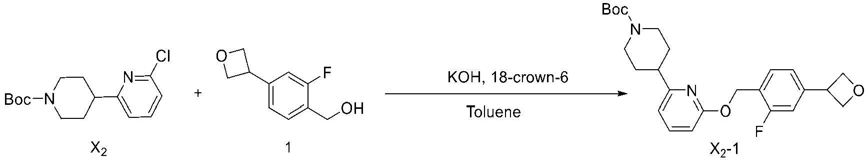

100 mL of dry toluene, 18 g of Compound X₂, 10 g of Compound 1 and 5.1 g of powdered potassium hydroxide were sequentially added to a reaction flask equipped with a water separator under nitrogen protection, heated up to 120°C and then stirred for reaction for 1 hour. After cooling to room temperature, 150 mL of ethyl acetate was added to dilute the reaction solution, and then 200 mL of purified water was added, stirred for 25 min, and allowed to stand for layer separation.

The organic phase was collected, and the aqueous phase was back-extracted once with 150 mL of ethyl acetate. The organic phases were combined, and washed with purified water and saturated brine separately (2×200 mL each), and the organic phase was collected. The organic phase was dried over anhydrous sodium sulfate, filtered, and concentrated to dryness.

### Post-treatment:

The concentrated dry crude product was added with 50 mL of ethyl acetate to dissolve, stirred for 1 h, and then filtered to remove insoluble substances. 100 mL of n-hexane was added to the filtrate for dilution. The organic phase above was poured into a pre-packed silica gel column, and the eluent was collected. The collected eluent was concentrated to dryness, added with a mixed solvent of methyl tert-butyl ether and n-hexane (V/V = 1:20) and heated to dissolve completely, then cooled to room temperature for crystallization, and filtered by suction. The filter cake was rinsed with n-hexane, and dried in vacuum to obtain Compound X₂-1 (13.5 g, a molar yield of 55%).

Another post-treatment method for use in Example 6 was as following:
The concentrated dry crude product was added with 150 mL of a mixed solvent of ethyl acetate and n-hexane (V/V = 1:2) to dissolve. The obtained organic phase was filtered through a suction funnel filled with silica gel (about 30 g), and the filter cake was rinsed with a mixed solvent of n-hexane and ethyl acetate (V/V = 1:2). The filtrate and rinse solution were combined, concentrated to dryness, then added with a mixed solvent of methyl tert-butyl ether and n-hexane (V/V = 1:4) and heated to dissolve completely, then cooled to room temperature for crystallization, and filtered by suction. The filter cake was rinsed with n-hexane, and dried in vacuum to obtain Compound X₂-1.

### Example 7: Preparation of the compound of Formula I

60 mL of DMF and 6.0 g of Compound XI were added to a reaction flask under nitrogen protection and stirred to dissolve completely. Subsequently, 3.5 g of sodium bicarbonate and 4.5 g of Compound V were added sequentially, stirred at room temperature for 60 minutes and then heated to 50 °C for a 6 h reaction. After the reaction solution was cooled to room temperature, purified water was added, and stirred for 2 hours before suction filtration. The filter cake was dissolved in ethyl acetate, and the organic phase was washed twice with saturated brine. The organic phase was dried over anhydrous sodium sulfate, filtered, and concentrated to dryness. The concentrated dry crude product was slurried with methyl tert-butyl ether at 50 °C for 2 h, then cooled to room temperature and subjected to suction filtration. The filter cake was rinsed with ethyl acetate and vacuum-dried to obtain Compound VI (a molar yield of 85%).

At room temperature, Compound VI and methanol were added to a reaction flask. Lithium hydroxide monohydrate was dissolved in purified water, and added to the reaction flask, stirred at room temperature for 1 h. The reaction solution was heated to 50 °C and stirred for 18 hours while maintaining the temperature. After the reaction solution was cooled to room temperature, purified water was added, and acetic acid was added dropwise to adjust the pH of the system to 5-6. The precipitated solid was collected, and then hot-slurried with methyl tert-butyl ether at 50 °C for 4 h, cooled to room temperature, and then stirred for 4 h. After suction filtration, the filter cake was rinsed with methyl tert-butyl ether. The obtained solid was vacuum-dried to yield Compound I (a molar yield of 90%).

The Examples above are presented merely for understanding the method and core concept of the present invention, but not for limiting the scope of the present invention. For those skilled in the art, any possible changes or substitutions made without departing from the concept of the present invention shall fall within the protection scope of the present invention.

## Claims

1. A method for preparing 2-(2-fluoro-4-(oxetan-3-yl)benzyl)oxy)-6-(piperidin-4-yl)pyridine (Formula XI), comprising the steps of:
a-1) dissolving Compound X in an organic solvent, adding Compound 1 to react in the presence of a base to obtain Compound X-1: 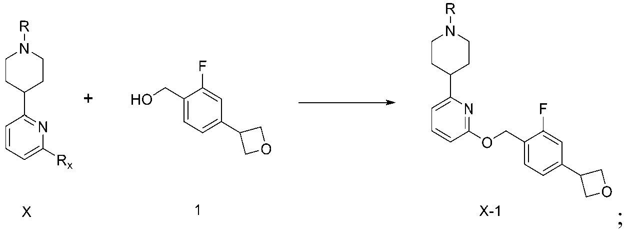
a-2) removing the amino protecting group R using a suitable solvent and suitable reaction conditions to obtain the Compound XI: 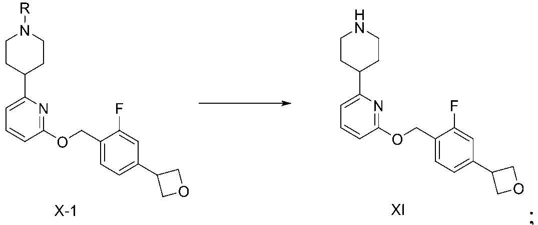
wherein said R is independently selected from the group consisting of benzyloxycarbonyl, tert-butyloxycarbonyl, p-methoxybenzyl, benzyl, trityl, p-toluenesulfonyl, phthaloyl and allyloxycarbonyl; and
said Rx is selected from the group consisting of Cl, Br and I, preferably Cl.

2. The method according to claim 1, wherein the organic solvent used in Step a-1) is an aprotic solvent selected from the group consisting of dioxane, N,N-dimethyl formamide, N,N-dimethylacetamide, dimethyl sulfoxide, acetonitrile, toluene, tetrahydrofuran, pyridine and 2-methyl tetrahydrofuran, preferably dioxane, or toluene;
and/or, the base used in Step a-1) is selected from the group consisting of sodium hydroxide, sodium bicarbonate, lithium hydroxide, potassium carbonate, potassium bicarbonate, potassium hydroxide, sodium methoxide, sodium tert-butoxide, potassium tert-butoxide, sodium ethoxide, n-butyl lithium, lithium diisopropylamide and lithium hexamethyldisilazide, preferably potassium tert-butoxide, or potassium hydroxide.

3. The method according to claim 1, wherein the molar feed ratio of the base to Compound 1 used in Step a-1) is 1:1 ~ 4:1, preferably 1:1 ~ 3:1;
and/or, the molar feed ratio of Compound X to Compound 1 in Step a-1) is 3:1 ~ 1:1;
and/or, after adding Compound X and Compound 1, the temperature can be controlled at T1 and then reduced to room temperature in Step a-1);
and/or, said T1 in Step a-1) is selected from 30 ~ 130°C, preferably 60 ~ 100°C.

4. The method according to claim 1, wherein the solvent used in Step a-2) is selected from the group consisting of dichloromethane, methanol, tetrahydrofuran, ethyl acetate, chloroform, acetonitrile and DMF, preferably dichloromethane;
and/or, the reaction temperature in Step a-2) is selected from -20 to 50°C.

5. A method for preparing (S)-2-((4-(6-((2-fluoro-4-(oxetan-3-yl)methyl)oxy)pyridin-2-yl)piperidin-1-yl)methyl)-1-(oxetan-2-ylmethyl)-1H-benzoimidazole-6-carboxylic acid (Formula I), comprising the steps of:
a-3) dissolving Compound XI in an organic solvent, adding a base and Compound V to react to obtain Compound VI: 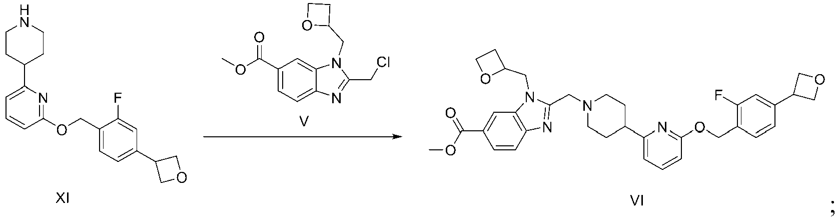
a-4) dissolving Compound VI in an organic solvent, adding a base to react to obtain Compound I:

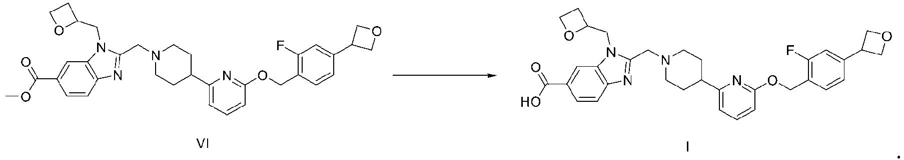

6. The method according to claim 5, wherein the organic solvent used in Step a-3) is selected from the group consisting of acetonitrile, N,N-dimethyl formamide, dimethyl sulfoxide, tetrahydrofuran, ethyl acetate, N,N-dimethyl formamide and n-hexane, preferably N,N-dimethyl formamide;
and/or, the molar feed ratio of the compound of Formula XI to the compound of Formula V in Step a-3) is 1:1 ~ 2:1;
and/or, the molar ratio of the base to Compound XI used in Step a-3) is 1:1 ~ 3:1.

7. The method according to claim 5, wherein the organic solvent used in Step a-4) is one or more selected from the group consisting of methanol, ethanol, isopropanol, n-butanol and tert-butanol, preferably methanol;
and/or, the molar ratio of the base to Compound VI used in Step a-4) is 1:1 ~ 5:1.

8. The method according to claim 1 and the method according to claim 5, wherein after the completion of the Step a-1), Step a-2), Step a-3) or Step a-4), post-reaction treatment can be optionally independently performed, wherein the post-treatment includes one or more of extraction, washing, drying, concentration and filtration.

9. A compound of Formula I prepared by the method according to claim 5.

10. The compound of Formula I according to claim 9 with a purity of more than 99%.

11. A compound of Formula XI prepared by the method according to claim 1.

12. The compound of Formula XI according to claim 11 with a purity of more than 98%.

13. Use of the method for preparing 2-(2-fluoro-4-(oxetan-3-yl)benzyl)oxy)-6-(piperidin-4-yl)pyridine (Formula XI) according to claim 1 in the preparation of the compound of Formula I, (S)-2-((4-(6-((2-fluoro-4-(oxetan-3-yl)methyl)oxy)pyridin-2-yl)piperidin-1-yl)methyl)-1-(oxetan-2-ylmethyl)-1H-benzoimidazole-6-carboxylic acid.

14. Use of the compound of Formula I prepared by the method according to claim 5, and the compound of Formula I according to claim 9 or 10, or a pharmaceutically acceptable salt thereof in the manufacture of a medicament for treating GLP-1 receptor mediated diseases or disorders and related diseases or disorders, wherein the GLP-1 receptor mediated diseases and related diseases are preferably selected from the group consisting of diabetes, hyperglycemia, insulin resistance, impaired glucose tolerance, diabetic nephropathy, diabetic neuropathy, diabetic retinopathy, adipocyte dysfunction, obesity, dyslipidemia, hyperinsulinemia, etc.; wherein the diabetes includes but is not limited to T1D and/or T2DM, idiopathic T1D, early-onset T2DM, latent autoimmune diabetes, atypical diabetes in adolescents, and gestational diabetes, etc.
